Europäisches Patentamt

European Patent Office

Office européen des brevets

(11) Publication number: **0 569 237 A2**

## EUROPEAN PATENT APPLICATION

(21) Application number: **93303514.9**

(22) Date of filing: **06.05.93**

(51) Int. Cl.5: **C12Q 1/70**, C12Q 1/68, C07H 21/04, C12P 19/34

The applicant has subsequently filed a sequence listing and declared, that it includes no new matter.

(30) Priority: **06.05.92 US 879684**

(43) Date of publication of application:
**10.11.93 Bulletin 93/45**

(84) Designated Contracting States:
**AT BE CH DE ES FR GB IT LI LU NL**

(71) Applicant: **GEN-PROBE INCORPORATED**
**9880 Campus Point Drive**
**San Diego California 92121-1514(US)**

(72) Inventor: **McDonough, Sherrol**
**4697 Robbins Street**
**San Diego, California 91222(US)**
Inventor: **Fultz, Timothy**
**358 Willow Creek Lane**
**Martinez, California 94553(US)**

(74) Representative: **Sexton, Jane Helen et al**
**J.A. Kemp & Co.**
**14 South Square**
**Gray's Inn**
**London WC1R 5LX (GB)**

(54) Nucleic acid amplification oligonucletodies and probes to human hepatitis B virus.

(57) Amplification oligonucleotides and hybridization assay probes specific for Human Hepatitis B Virus.

EP 0 569 237 A2

Field of the Invention

This invention relates to the design and construction of amplification oligonucleotides and probes to Human Hepatitis B Virus, which allow detection of the organism in a test sample.

Background of the Invention

Laboratory diagnosis of Hepatitis B Virus infection in humans is currently performed by demonstration of the presence of viral antigens (HBsAg, HBcAg and HBeAg), or their respective antibodies, in serum. Detection of Hepatitis B Virus DNA by nucleic acid hybridization is a more sensitive method for detection of virus in several clinical stages (Krogsgaard, Liver 8:257-283, 1988). Direct hybridization, however, lacks adequate sensitivity to detect HBV DNA in some patients, as shown by assay of patient samples following a nucleic acid amplification step such as the polymerase chain reaction ™ (See, Kaneko et al., 1989, Proc. Natl. Acad. Sci. U.S.A. 86:312-316; Larzul et al., 1988, J. Virol. Meth. 20:227-237; Sumazaki et al., 1989; J. Med. Virol. 27:304-308; and Theilman et al., 1989 Liver 9:322-328. Relevant references describing amplification primers and detection probes for Human Hepatitis B Virus include the following, none of which are admitted to be prior art to the claimed invention: Seelig et al., Deutsch Med Wochenschr 115:1307-1312, 1990; Brunetto et al., Proc. Natl. Acad. Sci., USA 88:4186-4190, 1991; Brunetto et al., Prog. Clin. Biol. Res. - (U.S.) 364:211-216. 1991; Fiordalisi et al., J. Med. Virol. 31:297-300, 1990; Liang et al., Hepatology 12(2)-204-212, 1990; Lo et al., J. Clin. Pathol. 42:840-846, 1989; Sumazaki et al., J. Med. Virol. 27:304-308, 1989; PCR protocols, 1990, chapter on HBV; Liaw et al., Hepatology 13(4):627-631, 1991; Pao et al., Am. J. Clin. Pathol. 95(4):591-596, 1991; Keller et al., J. Clin. Microbiol. 28(6):1411-1416, 1990; Pasquinelli et al., J. Med. Virol. 31:135-140, 1990; Musso, Lanthanide chelate-tagged nucleic acids probes, PCT/US88/03735; Urdea, Improved amplified nucleic acid hybridization assays for HBV, PCT/US90/02049; Urdea, et al. Gene 61:253-264, 1987; and Urdea, DNA-Dependent RNA polymerase transcripts as reporter molecules for signal amplification in nucleic acid hybridization assays, PCT/US91/00213.

Summary of the Invention

This invention discloses and claims novel amplification oligonucleotides and detection probes for the detection of Human Hepatitis B Virus. These probes detect unique sequences in the Human Hepatitis B Virus genome, and are capable of distinguishing between the Human Hepatitis B Virus and its known closest phylogenetic neighbors. These amplification oligonucleotides and probes may be used in an assay for the detection and/or quantitation of Human Hepatitis B Virus nucleic acid.

Thus, in a first aspect, the invention features hybridization assay probes able to distinguish Hepatitis B Virus from other viruses found in human blood or tissues, and amplification oligonucleotides able to selectively amplify Human Hepatitis B Virus nucleic acid. Specifically, the probe is a nucleotide polymer which hybridizes to the nucleic acid of HBV corresponding to (a) the 25 bases 403-427 of HBV sequence adw, (Ono et al. 1983, Nuc. Acids Res. 11(6):1747-1757, where base one is the first T in the EcoRI restriction endonuclease recognition sequence, (b) 22 bases corresponding to bases 1522-1543 of HBV adw sequence, or (c) 26 bases corresponding to bases 2367-2392 of HBV adw sequence, or a nucleotide polymer complementary thereto; i.e., the oligonucleotide comprises, consists essentially of, or consists of one of the sequences (reading 5' to 3')
(SEQ ID NO: 1) GAGGCATAGCAGCAGGATGAAGAGG,
(SEQ ID NO: 2) GGGCGCACCTCTCTTTACGCGG,
(SEQ ID NO: 3) GGTCCCCTAGAAGAAGAACTCCCTCG,
or oligonucleotides complementary thereto.

By "consists essentially of" is meant that the probe is provided as a purified nucleic acid which hybridizes under stringent hybridizing conditions with the desired target nucleic acid and not with other related targets in other virus nucleic acids, or in human nucleic acid. Such a probe may be linked to other nucleic acids which do not affect such hybridization. Generally, it is preferred that the probe be of between 15 and 100 (most preferably between 20 and 50) bases in size. It may, however, be provided in a vector.

In related aspects, the invention features a nucleotide polymer which specifically hybridizes under stringent hybridization conditions to the above oligonucleotides, a nucleic acid hybrid formed with the above oligonucleotides, and a nucleic acid sequence substantially complementary thereto. Such hybrids are useful because they allow the specific detection of viral nucleic acid, i.e., they represent the specific hybridization of probes of this invention with target nucleic acid.

In another aspect, the invention features amplification oligonucleotides useful for specific detection of Human Hepatitis B Virus in an amplification assay. The amplification oligonucleotides are complementary to a conserved region of HBV genomic nucleic acid and are nucleotide polymers able to hybridize to the nucleic acid of HBV corresponding to HBV sequence adw bases 365 to 389, bases 455 to 479, bases 466 to 490, bases or corresponding bases 1415 to 1436, bases 1557 to 1587, bases 2301 to 2333, bases 2418 to 2442, or bases 2421 to 2444.

Specifically, such oligonucleotides consist, comprise or consist essentially of these selected from (reading 5' to 3'):

SEQ. ID. NO. 4:(X)GGTTATCGCTGGATGTGTCTGCGGC,

SEQ. ID. NO. 5:(X)GAGGACAAACGGGCAACATACCTTG,

SEQ. ID. NO. 6:(X)TCCTGGAATTAGAGGACAAACGGGC,

SEQ. ID. NO. 7:(X)TCCTGGAATTAGAGGATAAACGGGC,

SEQ. ID. NO. 8:(X)CGTCCTTTGTTTACGTCCCGTC,

SEQ. ID. NO. 9:(X)GCACACGGACCGGCAGATGAGAAGGC,

SEQ. ID. NO. 10:(X)CACCAAATGCCCCTATCTTATCAACACTTCCGG,

SEQ. ID. NO. 11:(X)CCCGAGATTGAGATCTTCTGCGAC, and

SEQ. ID. NO. 12:(X)CGAGATTGAGATCTTCTGCGACGCG

where (X) is nothing or a 5' oligonucleotide sequence that is recognized by an enzyme RNA polymerase (including but not limited to the promoter sequence for T7, T3, or SP6 RNA polymerase), which enhances initiation or elongation of RNA transcription by an RNA polymerase. One example of X includes the sequence (SEQ. ID. NO. 13) 5'-AATTTAATACGACTCACTATAGGGAGA-3'.

These amplification oligonucleotides are used in a nucleic acid amplification assay such as the polymerase chain reaction or an amplification reaction using RNA polymerase, DNA polymerase and RNaseH or its equivalent, as described by Kacian and Fultz, supra, and by Sninsky et al. US. Patent No. 5,079,351, both hereby incorporated by reference herein.

The amplification oligonucleotides and probes of this invention offer a rapid, non-subjective method of identification and quantitation of a sample for specific sequences unique to all strains of HBV.

Other features and advantages of the invention will be apparent from the following description of the preferred embodiments thereof, and from the claims.

Description of the Preferred Embodiments

We have discovered particularly useful DNA probes complementary to particular nucleic acid sequences of Human Hepatitis B Virus. Furthermore, we have successfully used those probes in a specific assay for the detection of Human Hepatitis B Virus, distinguishing it from the known and presumed most closely related taxonomic or phylogenetic neighbors found in human blood or tissues.

We have also identified particularly useful amplification oligonucleotides which are complementary to the Human Hepatitis B Virus nucleic acid, and have used these oligonucleotides, e.g., as primers or promoter-primer combinations (i.e., a primer having a promoter sequence attached), to amplify the nucleic acid of Human Hepatitis B Virus, allowing its direct detection in a serum sample.

Useful guidelines for designing amplification oligonucleotides and probes with desired characteristics are described herein. The optimal sites for amplifying and probing contain two, and preferably three, conserved regions greater than about 15 bases in length, within about 350 bases, and preferably within 150 bases, of contiguous sequence. The degree of amplification observed with a set of primers or primer/splice templates depends on several factors, including the ability of the oligonucleotides to hybridize to their complementary sequences and their ability to be extended enzymatically. Because the extent and specificity of hybridization reactions are affected by a number of factors, manipulation of those factors will determine the exact sensitivity and specificity of a particular oligonucleotide, whether perfectly complementary to its target or not. The importance and effect of various assay conditions are known to those skilled in the art as described in Hogan et al. Nucleic Acid Probes for Detection and/or Quantitation of non-Viral Organisms. U.S. Serial No. 07/806,929, filed December 11, 1991, and Milliman, Nucleic Acid Probes to Haemophilus influenzae, U.S. Serial No. 07/690,788, filed 4/25/91 assigned to the same assignee as the present application and hereby incorporated by reference herein.

The length of the target nucleic acid sequence and, accordingly, the length of the probe sequence can be important. In some cases, there may be several sequences from a particular region, varying in location and length, which will yield probes with the desired hybridization characteristics. In other cases, one sequence may be significantly better than another which differs merely by a single base. While it is possible for nucleic acids that are not perfectly complementary to hybridize, the longest stretch of perfectly homologous base sequence will normally primarily determine hybrid stability. While oligonucleotide probes of different lengths and base composition may be used, oligonucleotide probes preferred in this invention are between about 10 to 50 bases in length and are sufficiently homologous to the target nucleic acid. We have found that optimal primers have target-binding regions of 18-28 bases, with a predicted Tm to target of 65°C.

Amplification oligonucleotides or probes should be positioned so as to minimize the stability of the oligomer: nontarget (i.e., nucleic acid with similar sequence to target nucleic acid) nucleic acid hybrid. It is preferred that the amplification oligomers and detection probes are able to distinguish between target and non-target sequences. In designing probes, the differences in these Tm values should be as large as possible (e.g., at least 2°C and preferably 5°C).

Regions of the nucleic acid which are known to form strong internal structures inhibitory to hybridization are less preferred. Likewise, probes and amplification oligonucleotides with extensive self-complementarity should be avoided.

The degree of non-specific extension (primer-dimer or non-target copying) can also affect amplification efficiency, therefore primers are selected to have low self- or cross-complementarity, particularly at the 3' ends of the sequence. Long homopolymer tracts and high GC content are avoided to reduce spurious primer extension. Computer programs are available to aid in this aspect of the design.

Hybridization is the association of two single strands of complementary nucleic acid to form a hydrogen bonded double strand. It is implicit that if one of the two strands is wholly or partially involved in a hybrid that it will be less able to participate in formation of a new hybrid. By designing a probe so that a substantial portion of the sequence of interest is single stranded, the rate and extent of hybridization may be greatly increased. If the target is an integrated genomic sequence then it will naturally occur in a double stranded form, as is the case with the product of the polymerase chain reaction (PCR). These double stranded targets are naturally inhibitory to hybridization with a probe and require denaturation prior to the hybridization step. Probes of this invention are directed to the major product of an amplification system, which is single-stranded RNA. Finally, there can be intramolecular and intermolecular hybrids formed within a probe if there is sufficient self complementarity. Such structures can be avoided through careful probe design. Computer programs are available to search for this type of interaction.

Once synthesized, selected oligonucleotides may be labelled by any of several well known methods. Sambrook et al., 2 Molecular Cloning 11 (2d ed. 1989). Useful labels include radioisotopes as well as non-radioactive reporting groups. We currently prefer to use acridinium esters.

Oligonucleotide/target hybrid melting temperature may be determined by isotopic methods well known to those skilled in the art. It should be noted that the Tm for a given hybrid will vary depending on the hybridization solution being used. Sambrook et al., supra.

Rate of hybridization may be measured by determining the $C_0t_{\frac{1}{4}}$. The $C_0t_{\frac{1}{4}}$ is found graphically by standard procedure.

The following example sets forth oligonucleotide probes complementary to a unique nucleic acid sequence from a target organism, and their use in a hybridisation assay.

Example:

Probes specific for Human Hepatitis B Virus were identified by comparison of sequences obtained from the published database GenBank. The following sequences were characterized and shown to be specific for Human Hepatitis B Virus;
(SEQ ID NO: 1) GAGGCATAGCAGCAGGATGAAGAGG (probe 1)
(SEQ ID NO: 2) GGGCGCACCTCTCTTTACGCGG (probe 2)
(SEQ ID NO: 3) GGTCCCCTAGAAGAAGAACTCCCTCG (probe 3)
Phylogenetically near neighbors including Woodchuck Hepatitis Virus and Ground Squirrel Hepatitis Virus were used as comparisons with the sequence of Human Hepatitis B Virus.

To demonstrate the reactivity and specificity of the probes for Human Hepatitis B Virus, they were used in a hybridization assay. The probes were first synthesized with a non-nucleotide linker, then labelled with a chemiluminescent acridinium ester as described in EPO Patent Application No. PCT/US88/03361, entitled "Acridinium Ester Labeling and Purification of Nucleotide Probes filed October 5, 1988. The acridinium ester attached to unhybridized probe is rendered non-chemiluminescent under mild alkaline conditions, while the acridinium ester attached to hybridized probe is relatively resistant. Thus, it is possible to assay for hybridization of acridinium ester-labelled probe by incubation with an alkaline buffer, followed by detection of chemiluminescence in a luminometer. Results are given in RLU, the quantity of photons emitted by the labelled-probe measured by the luminometer The conditions of hybridization, hydrolysis and

4

detection are described in Arnold, et al., 35 Clin. Chem. 1588, 1989.

In the following experiment, DNA prepared from clones containing full or partial sequences of the viruses was assayed. An example of such a method is provided by Sambrook et al., supra. The source of DNA for the clones was as follows; Human Hepatitis B Virus serotype ADW, obtained from ATCC #45020; Human Immunodeficiency Virus type 1, BH10 (Ratner et al., Nature 312:277-284. 1985); Human Immunodeficiency Virus type 2 NIHZ (Zagury et al., Proc. Natl. Acad. Sci. USA 85:5941-5945. 1988), Human T-cell leukemia virus type 1 pMT-2, (Clarke et al., Nature 305:60-62. 1983); and Human T-cell leukemia virus type 2 (Shimotohmo et al., Proc. Natl. Acad. Sci. U.S.A. 82:3101-3105. 1985). An RLU value greater than 3,000 RLU was a positive result; less than 3,000 RLU was a negative result.

The following data show that the probes do not cross react with DNA from closely related viruses found in human blood or tissues. The samples were also tested with a probe specific to each target. A positive signal from this probe provided confirmation of sample adequacy.

| Target | Probe 1 | Probe 2 | Probe 3 | Positive Control |
|---|---|---|---|---|
| HBV ADW 1.3, 1.8 kb | | | | |
| BamHI fragments | 11,789 | 168,795 | 18,982 | --- |
| HIV-1 9 kb SstI fragment | 429 | 2,788 | 272 | 98,314 |
| HIV-2 9 kb SstI fragment | 594 | 2,287 | 272 | 33,635 |
| HTLV-1 5' 4.6 kb | | | | |
| SstI-BamHI fragment | 413 | 365 | 148 | 24,999 |
| HTLV-1 3' 4.4 kb | | | | |
| XbaI-SstI fragment | 421 | 341 | 145 | 27,149 |
| HTLV-2 3.5 kb BamHI fragment | 554 | 324 | 153 | 47,879 |
| HTLV-2 5 kb BamHI fragment | 359 | 328 | 155 | 16,690 |

The above data confirm that the novel probes are capable of distinguishing Human Hepatitis B Virus from these viruses found in human serum.

To demonstrate the reactivity of the primers and probes for Human Hepatitis B virus, the following experiment was performed. Plasmid DNA containing the Human Hepatitis B Virus sequence was linearized with a restriction endonuclease, and added to amplification reactions using standard polymerase chain reaction conditions. Following amplification, the products were analyzed by hybridization protection assay.

| Primers | Target Level (Units) | RLU |
|---|---|---|
| Seq ID 4/7 | 1,000 | 25,737 |
| | 0 | 474 |
| Seq ID 8/9 | 1,000 | 36,278 |
| | 0 | 904 |
| Seq ID 10/12 | 1,000 | 152,306 |
| | 0 | 797 |

To show that the amplification oligomers also work in a transcription based amplification assay, patient samples were evaluated. Ten microliter plasma samples from patients (uninfected are termed "normal") were treated with ten microliters of 0.2 N KOH at 95°C for 15 minutes, then neutralized. A buffer containing reagents for amplification was added to a final concentration of 150 nM each primer, 88 mM imidazole, 55 mM glutamate, 2.5% PVP-40, 2 mM spermidine, 15 mM N-acetyl-cysteine, 6.25 mM rATP and rGTP, 2.5 mM rCTP and rUTP, 0.2 mM each of dTTP, dATP, dCTP and dGTP, 21 mM $MgCl_2$, 0.5 mM zinc acetate,

5

0.005% Triton X-100, 1200 U of MMLV reverse transcriptase, 200 U of T7 RNA polymerase and incubated at 36°C for 3 hours. The entire reaction was analyzed by Hybridization Protection Assay as described above.

The results, in Relative Light Units, or RLU, are shown.

| Primers: | SEQ ID 4/7 | SEQ ID 8/9 | SEQ ID 10/12 |
|---|---|---|---|
| Probe: | 1 | 2 | 3 |
| Sample | | | |
| #1 | 221,521 | 22,981 | 1,151,234 |
| #2 | 243,659 | 580,709 | 1,544,800 |
| #3 | 283,827 | 119,153 | 1,526,431 |
| #4 | 12,988 | 7,953 | 749,369 |
| #5 | 277,533 | 145,727 | 1,502,496 |
| #6 | 266,310 | 107,026 | 1,458,598 |
| #7 | 245,661 | 142,530 | 1,504,906 |
| #8 | 301,941 | 149,795 | 1,455,712 |
| Normal #1 | 7,294 | 9,303 | 6,271 |
| Normal #2 | 6,590 | 6,164 | 4,404 |

The results shown are the average of three replicates. All of the positive clinical samples were detected with two of the primer pairs. The third primer pair detected all but one sample which contained low amounts of target. Optimization of amplification conditions is expected to increase RLU observed with each primer pair. Both normal serum samples gave <10,000 RLU in the assay.

Other embodiments are within the following claims.

## Claims

1.  An oligonucleotide consisting essentially of the sequence shown as SEQ. ID. NO. 1, or an oligonucleotide complementary thereto.

2.  An oligonucleotide consisting essentially of the sequence shown as SEQ. ID. NO. 2, or an oligonucleotide complementary thereto.

3.  A nucleic acid hybrid formed between an oligonucleotide consisting essentially of the sequence shown as SEQ. ID. NO. 1 or an oligonucleotide complementary thereto, and a nucleic acid sequence substantially complementary to said oligonucleotide.

4. A nucleic acid hybrid formed between an oligonucleotide consisting essentially of the sequence shown as SEQ. ID. NO. 2 or an oligonucleotide complementary thereto, and a nucleic acid sequence substantially complementary to said oligonucleotide.

5. An oligonucleotide selected from the group consisting of:
(X)GGTTATCGCTGGATGTGTCTGCGGC,
(X)GAGGACAAACGGGCAACATACCTTG,
(X)TCCTGGAATTAGAGGACAAACGGGC,
(X)TCCTGGAATTAGAGGATAAACGGGC,
(X)CGTCCTTTGTTTACGTCCCGTC,
(X)GCACACGGACCGGCAGATGAGAAGGC, and
(X)CGAGATTGAGATCTTCTGCGACGCG,
where X is nothing or comprises an oligonucleotide sequence recognized by an RNA polymerase or which enhances initiation or elongation by an RNA polymerase.

6. A kit comprising two oligonucleotides selected from the group consisting of;
(X)GGTTATCGCTGGATGTGTCTGCGGC,
(X)GAGGACAAACGGGCAACATACCTTG,
(X)TCCTGGAATTAGAGGACAAACGGGC,
(X)TCCTGGAATTAGAGGATAAACGGGC,
(X)CGTCCTTTGTTTACGTCCCGTC,
(X)GCACACGGACCGGCAGATGAGAAGGC,
(X)CGAGATTGAGATCTTCTGCGACGCG,
where X is nothing or comprises an oligonucleotide sequence recognized by an RNA polymerase or which enhances initiation or elongation by an RNA polymerase.

7. A kit comprising oligonucleotides having the following sequences;
(X)GGTTATCGCTGGATGTGTCTGCGGC,
(X)TCCTGGAATTAGAGGACAAACGGGC, and
GAGGCATAGCAGCAGGATGAAGAGG,
where X is nothing or comprises an oligonucleotide sequence recognized by an RNA polymerase or which enhances initiation or elongation by an RNA polymerase.

8. A kit comprising oligonucleotides having the following sequences;
(X)CGTCCTTTGTTTACGTCCCGTC,
(X)GCACACGGACCGGCAGATGAGAAGGC, and
GGGCGCACCTCTCTTTACGCGG,
where X is nothing or comprises an oligonucleotide sequence recognized by an RNA polymerase or which enhances initiation or elongation by an RNA polymerase.

9. A method for selectively amplifying Human Hepatitis B Virus nucleic acid in a sample, comprising the step of amplifying said nucleic acid with one or more oligonucleotides selected from the group consisting of;
(X)GGTTATCGCTGGATGTGTCTGCGGC,
(X)GAGGACAAACGGGCAACATACCTTG,
(X)TCCTGGAATTAGAGGACAAACGGGC,
(X)TCCTGGAATTAGAGGATAAACGGGC,
(X)CGTCCTTTGTTTACGTCCCGTC,
(X)GCACACGGACCGGCAGATGAGAAGGC, and
(X)CGAGATTGAGATCTTCTGCGACGCG; where X is nothing or comprises an oligonucleotide sequence recognized by an RNA polymerase or which enhances initiation or elongation by an RNA polymerase.

10. A method for detecting Human Hepatitis B Virus nucleic acid in a sample comprising the step of hybridizing nucleic acid obtained directly or amplified from said sample with an oligonucleotide selected from the group consisting of GAGGCATAGCAGCAGGATGAAGAGG, and GGGCGCACCTCTCTTTACGCGG.

11. A method for detecting Human Hepatitis B Virus nucleic acid comprising the step of amplifying said nucleic acid with the oligonucleotide polymers
(X)GGTTATCGCTGGATGTGTCTGCGGC and
(X)TCCTGGAATTAGAGGACAAACGGGC, and detecting the amplified nucleic acid with an oligonucleotide comprising the sequence GAGGCATAGCAGCAGGATGAAGAGG; where X is nothing or comprises an oligonucleotide sequence recognized by an RNA polymerase or which enhances initiation or elongation by an RNA polymerase.

12. A method for detecting Human Hepatitis B Virus nucleic acid comprising the step of amplifying said nucleic acid with the oligonucleotide polymers
(X)CGTCCTTTGTTTACGTCCCGTC and
(X)GCACACGGACCGGCAGATGAGAAGGC,
and detecting the amplified nucleic acid with an oligonucleotide comprising the sequence GGGCGCACCTCTCTTTACGCGG; where X is nothing or comprises an oligonucleotide sequence recognized by an RNA polymerase or which enhances initiation or elongation by an RNA polymerase.

13. A method for selectively amplifying Human Hepatitis B Virus nucleic acid in a sample comprising amplifying nucleic acid corresponding to bases 365 to 490 of HBV adw sequence.

14. A method for selectively amplifying Human Hepatitis B Virus nucleic acid in a sample comprising amplifying nucleic acid corresponding to bases 1415 to 1587 of HBV adw sequence.

15. A method for selectively amplifying Human Hepatitis B Virus nucleic acid in a sample comprising amplifying nucleic acid corresponding to bases 2301 to 2442 or 2301 to 2444 of HBV adw sequence.

(1) GENERAL INFORMATION:

(i) APPLICANTS:    GEN-PROBE INCORPORATED

(ii) TITLE OF INVENTION:   NUCLEIC ACID AMPLIFICATION OLIGONUCLEOTIDES and PROBES TO HUMAN HEPATITIS B VIRUS

(iii) NUMBER OF SEQUENCES:   13

(1)  INFORMATION FOR SEQ ID NO:     1:

        (i)  SEQUENCE CHARACTERISTICS:

                (A)  LENGTH:                    25
                (B)  TYPE:                      nucleic acid
                (C)  STRANDEDNESS:              single
                (D)  TOPOLOGY:                  linear

        (ii)  SEQUENCE DESCRIPTION : SEQ ID NO: 1:

GAGGCATAGC AGCAGGATGA AGAGG            25


(2)  INFORMATION FOR SEQ ID NO:     2:

        (i)  SEQUENCE CHARACTERISTICS:

                (A)  LENGTH:                    22
                (B)  TYPE:                      nucleic acid
                (C)  STRANDEDNESS:              single
                (D)  TOPOLOGY:                  linear

        (ii)  SEQUENCE DESCRIPTION : SEQ ID NO: 2:

GGGCGCACCT CTCTTTACGC GG            22

(3)  INFORMATION FOR SEQ ID NO:     3:

        (i)  SEQUENCE CHARACTERISTICS:

                (A)  LENGTH:                    26
                (B)  TYPE:                      nucleic acid
                (C)  STRANDEDNESS:              single
                (D)  TOPOLOGY:                  linear

        (ii)  SEQUENCE DESCRIPTION : SEQ ID NO: 3:

GGTCCCCTAG AAGAAGAACT CCCTCG          26


(4) INFORMATION FOR SEQ ID NO:    4:

      (i) SEQUENCE CHARACTERISTICS:

          (A) LENGTH:              52
          (B) TYPE:                nucleic acid
          (C) STRANDEDNESS:        single
          (D) TOPOLOGY:            linear

      (ii) SEQUENCE DESCRIPTION : SEQ ID NO: 4:

AATTTAATAC GACTCACTAT AGGGAGAGGT TATCGCTGGA TGTGTCTGCG GC          52


(5) INFORMATION FOR SEQ ID NO:    5:

      (i) SEQUENCE CHARACTERISTICS:

          (A) LENGTH:              25
          (B) TYPE:                nucleic acid
          (C) STRANDEDNESS:        single
          (D) TOPOLOGY:            linear

      (ii) SEQUENCE DESCRIPTION : SEQ ID NO: 5:

GAGGACAAAC GGGCAACATA CCTTG          25

(6) INFORMATION FOR SEQ ID NO:    6:

      (i) SEQUENCE CHARACTERISTICS:

          (A) LENGTH:              52
          (B) TYPE:                nucleic acid
          (C) STRANDEDNESS:        single
          (D) TOPOLOGY:            linear

      (ii) SEQUENCE DESCRIPTION : SEQ ID NO: 6:

AATTTAATAC GACTCACTAT AGGGAGATCC TGGAATTAGA GGACAAACGG GC    52


(7) INFORMATION FOR SEQ ID NO:    7:

      (i) SEQUENCE CHARACTERISTICS:

```
                    (A)  LENGTH:                25
                    (B)  TYPE:                  nucleic acid
                    (C)  STRANDEDNESS:          single
                    (D)  TOPOLOGY:              linear

        (ii)  SEQUENCE DESCRIPTION : SEQ ID NO: 7:

TCCTGGAATT AGAGGATAAA CGGGC            25


(8)  INFORMATION FOR SEQ ID NO:     8:

        (i)  SEQUENCE CHARACTERISTICS:

                    (A)  LENGTH:                22
                    (B)  TYPE:                  nucleic acid
                    (C)  STRANDEDNESS:          single
                    (D)  TOPOLOGY:              linear

        (ii)  SEQUENCE DESCRIPTION : SEQ ID NO: 8:

CGTCCTTTGT TTACGTCCCG TC              22

(9)  INFORMATION FOR SEQ ID NO:     9:

        (i)  SEQUENCE CHARACTERISTICS:

                    (A)  LENGTH:                53
                    (B)  TYPE:                  nucleic acid
                    (C)  STRANDEDNESS:          single
                    (D)  TOPOLOGY:              linear

        (ii)  SEQUENCE DESCRIPTION : SEQ ID NO: 9:

AATTTAATAC GACTCACTAT AGGGAGAGCA CACGGACCGG CAGATGAGAA GGC          53


(10) INFORMATION FOR SEQ ID NO:    10:

        (i)  SEQUENCE CHARACTERISTICS:

                    (A)  LENGTH:                33
                    (B)  TYPE:                  nucleic acid
                    (C)  STRANDEDNESS:          single
                    (D)  TOPOLOGY:              linear
```

9

(ii) SEQUENCE DESCRIPTION : SEQ ID NO: 10:

CACCAAATGC CCCTATCTTA TCAACACTTC CGG          33

(11) INFORMATION FOR SEQ ID NO:     11:

    (i) SEQUENCE CHARACTERISTICS:

        (A) LENGTH:          51
        (B) TYPE:            nucleic acid
        (C) STRANDEDNESS:    single
        (D) TOPOLOGY:        linear

    (ii) SEQUENCE DESCRIPTION : SEQ ID NO: 11:

AATTTAATAC GACTCACTAT AGGGAGACCC GAGATTGAGA TCTTCTGCGA C          51

(12) INFORMATION FOR SEQ ID NO:     12:

    (i) SEQUENCE CHARACTERISTICS:

        (A) LENGTH:          57
        (B) TYPE:            nucleic acid
        (C) STRANDEDNESS:    single
        (D) TOPOLOGY:        linear

    (ii) SEQUENCE DESCRIPTION : SEQ ID NO: 12:

AATTTAATAC GACTCACTAT AGGGAGACCA CACGAGATTG AGATCTTCTG CGACGCG          57

(12) INFORMATION FOR SEQ ID NO:     13:

    (i) SEQUENCE CHARACTERISTICS:

        (A) LENGTH:          27
        (B) TYPE:            nucleic acid
        (C) STRANDEDNESS:    single
        (D) TOPOLOGY:        linear

    (ii) SEQUENCE DESCRIPTION : SEQ ID NO: 13:

AATTTAATAC GACTCACTAT AGGGAGA          27